(19) Europäisches Patentamt
European Patent Office
Office européen des brevets

(11) **EP 1 983 049 B1**

(12) **EUROPÄISCHE PATENTSCHRIFT**

(45) Veröffentlichungstag und Bekanntmachung des Hinweises auf die Patenterteilung:
**22.12.2010 Patentblatt 2010/51**

(51) Int Cl.:
*C12N 15/09* (2006.01)   *C07K 7/08* (2006.01)
*A61K 38/10* (2006.01)

(21) Anmeldenummer: **08009541.7**

(22) Anmeldetag: **11.05.2006**

(54) **An die Lck-SH3 und Hck-SH3 Domäne bindende Peptide**

Peptides binding to the Lck-SH3 and Hck-SH3 domains

Peptides liant les domaines Lck-SH3 et Hck-SH3

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.05.2005 DE 102005023761**

(43) Veröffentlichungstag der Anmeldung:
**22.10.2008 Patentblatt 2008/43**

(62) Dokumentnummer(n) der früheren Anmeldung(en) nach Art. 76 EPÜ:
**06009706.0 / 1 724 346**

(73) Patentinhaber: **Forschungszentrum Jülich GmbH 52425 Jülich (DE)**

(72) Erfinder:
  • **Willbold, Dieter**
    **52428 Jülich (DE)**
  • **Hoffmann, Silke**
    **61250 Usingen (DE)**
  • **Wiesehan, Katja**
    **52428 Jülich (DE)**
  • **Tran, Thi Tuyen**
    **48231 Warendorf (DE)**

(56) Entgegenhaltungen:
  • **SCHWEIMER KRISTIAN ET AL: "Structural investigation of the binding of a herpesviral protein to the SH3 domain of tyrosine kinase Lck" BIOCHEMISTRY, Bd. 41, Nr. 16, 23. April 2002 (2002-04-23), Seiten 5120-5130, XP002398780 ISSN: 0006-2960**

  • **HORITA DAVID A ET AL: "Solution structure of the human Hck SH3 domain and identification of its ligand binding site" JOURNAL OF MOLECULAR BIOLOGY, Bd. 278, Nr. 1, 24. April 1998 (1998-04-24), Seiten 253-265, XP004453992 ISSN: 0022-2836**

  • **GREENWAY ALISON ET AL: "Human immunodeficiency virus type 1 Nef binds directly to Lck and mitogen-activated protein kinase, inhibiting kinase activity" JOURNAL OF VIROLOGY, Bd. 70, Nr. 10, 1996, Seiten 6701-6708, XP002398781 ISSN: 0022-538X**

  • **ALEXANDROPOULOUS KONSTANTINA ET AL: "Proline-rich sequences that bind to Src homology 3 domains with individual specificities" PROCEEDINGS OF THE NATIONAL ACADEMY OF SCIENCES OF THE UNITED STATES OF AMERICA, Bd. 92, Nr. 8, 1995, Seiten 3110-3114, XP002398782 ISSN: 0027-8424**

  • **BRIGGS SCOTT D ET AL: "The Ras GTPase-activating Protein (GAP) Is an SH3 Domain-binding Protein and Substrate for the Src-related Tyrosine Kinase, Hck" JOURNAL OF BIOLOGICAL CHEMISTRY, Bd. 270, Nr. 24, 1995, Seiten 14718-14724, XP002398783 ISSN: 0021-9258**

  • **LEE C-H ET AL: "A single amino acid in the SH3 domain of Hck determines its high affinity and specificity in binding to HIV-1 Nef protein" EMBO JOURNAL, OXFORD UNIVERSITY PRESS, SURREY, GB, Bd. 14, Nr. 20, 1995, Seiten 5006-5015, XP002236097 ISSN: 0261-4189**

  • **TRAN TUYEN ET AL: "Insights into human Lck SH3 domain binding specificity: Different binding modes of artificial and native ligands" BIOCHEMISTRY, Bd. 44, Nr. 45, November 2005 (2005-11), Seiten 15042-15052, XP002430256 ISSN: 0006-2960**

SEQUENCE LISTING

<110> Forschungszentrum Jülich GmbH

<120> An die Lck-SH3 und Hck-sH3 Domäne bindende Peptide

<130> Pol. 2218a

<160> 4

<170> PatentIn version 3.4

<210>
<211> 12
<212> PRT
<213> Artificial Sequence

<220>
<223> Die Sequenz wurde aus einer Phagenbibliothek erhalten

<400> 1

His Ser Lys Tyr Pro Leu Pro Pro Leu Pro Ser Leu
1               5               10

<210> 2
<211> 12
<212> PRT
<213> Artificial Sequence

<220>
<223> Die Sequenz wurde aus einer Phagenbibliothek erhalten

<400> 2

```
His Ser Lys Arg Pro Leu Pro Pro Leu Pro Ser Leu
 1               5               10
```

<210> 3
<211> 36 <212> DNA
<213> Artificial Sequence

<220>
<223> Die Sequenz entspricht der der Sequenz Nr.1 zugeordneten DNA Sequenz

<400> 3
cattctaagt atccgctgcc tccgttgccg tctctt          36

<210> 4
<211> 12
<212> PRT
<213> Artificial Sequence

<220>
<223> Die Sequenz wurde aus einer Phagenbibliothek erhalten

<400> 4

```
His Phe Lys Arg Pro Leu Pro Pro Leu Pro Ser Leu
 1               5               10
```

## Beschreibung

Cytoplasmatische Tyrosin-spezifische Proteinkinasen (PTKs) sind intrazelluläre Effektormoleküle, die an verschiedenen Signalübertragungswegen beteiligt sind.

PTKs können dabei über ihre nicht-katalytischen- SH3- und SH2-Domänen mit bestimmten Subtratproteinen assoziieren und diese dann im Sinne einer Signalweitergabe durch eine Tyrosinphosphorylierung aktivieren. Die menschliche T-Zell Tyrosinkinase (Lck) nimmt eine Schlüsselrolle bei der Signalübertragung in T-Zellen ein und ist essentiell für die Entwicklung und Funktion dieser Zellen. Krankheiterreger, wie das menschliche Immunschwächevirus (HIV) oder Herpesvirus saimiri, haben Effektormoleküle entwickelt, die mit Lck wechselwirken, eine geregelte Immunantwort verhindern und so z. B. ihre eigene Vermehrung gewährleisten. Die Bindung der viralen Proteine an die Lck wird dabei durch die Interaktion einer Prolin-reichen Region (PPHII) im Virusprotein mit der SH3-Domäne der Lck vermittelt.

Von einer großen Anzahl von SH3 Domänen wurde die Spezifität mittlerweile mittels Phagendisplay oder anderen Methoden von verschiedenen Arbeitsgruppen untersucht. Beispielhaft kann die Veröffentlichung von Sparks AB, Rider JE, Hoffmann NG, Fowlkes DM, Quillam LA, Kay BK "Distict ligand preferences of Src homology 3 domains from Src, Yes, Abl, Cortactin, p53bp2, PLCgamma, Crk and Grb2" genannt werden. Die Ergebnisse dieser Arbeiten sowie die Ergebnisse sehr aufwendiger, systematischer Arbeiten an ganzen Genomen (z. B. Hefe) haben aber bis zum heutigen Tage zu keinem verlässlichen Erkennungscode für SH3-Domänen geführt, dass heißt, es besteht bisher keine Möglichkeit systematisch zu erkennen, welche Polyprolinhaltige Peptide an die SH3-Domäne der Lck binden. Allerdings sind Peptide aus natürlichen Liganden der Lck SH3 bekannt, die an die Lck-SH3 binden, wie in Poghosyan Z. et al. in Phosphorylation-dependent interactions between ADAM15 cytoplasmic domain and Scr family protein-tyrosine kinase J Biol Chem. 2002 Feb 15; 277(7):4999-5007 veröffentlicht.

In der Veröffentlichung "Structural Investigation of the Binding of a Herpesviral Protein to the SH3 Domain of Tyrosine Kinase Lck" von Kristian Schweimer et al. in Biochemistry 2002, 41, 5120 - 5230 werden Petide, die von dem Tyrosinkinase interagierenden Protein aus Herpesvirus saimiri hergeleitet sind offenbart, die mit der SH3 Domäne von Lck und der SH3 Domäne von Hck interagieren.

Es ist die Aufgabe der Erfindung, Liganden zu identifizieren, die eine möglichst affine Bindung an die Lck-SH3 Domäne eingehen und vorzugsweise in der Lage sind, die Bindung z. B. viraler Effektormoleküle an die Lck-SH3 zu unterbinden oder eine schon erfolgte Bindung wieder aufzulösen. Weiterhin soll ein Wirkstoff zur Verfügung gestellt werden, welcher dazu in der Lage ist, die Immunabwer zu beeinflussen.

Erfindungsgemäß wird die Aufgabe durch die Bereitstellung des in dem Sequenzprotokoll 2 angegebenen Peptids und dessen Verwendung zur Besetzung der SH3-Domäne von Tyrosinkinase (Lck) von Immunzellen gelöst.

Erfindungsgemäß wird die SH3-Domäne von Lck mit dem erfindungsgemäßen Peptid nach Sequenzprotokoll 2 mit einer hohen Affinität besetzt.

Das Peptid gemäß Seq. 2 bindet zusätzlich an die Hck-SH3 Domäne, was den Vorteil hat, die Bindung z. B. viraler Effektormoleküle an die Hck-SH3 zu unterbinden oder eine solche Bildung aufzulösen.

Das erfindungsgemäße Peptid bindet besonders bevorzugt an die Lck- SH3-Domänen vom Menschen (Homo sapiens), von der Maus (Mus musculus), der Ratte (Rattus norvegicus), dem Hund (Canis familiaris), dem Nachtaffe (Aotus nancymaae) oder dem Schimpansen (Pan troglodytes) an. Allerdings ist die Anbindung nicht auf diese Spezies beschränkt.

Weiterhin bindet das erfindungsgemäße Peptid beispielhaft, aber nicht beschränkend an die Hck-SH3-Domäne vom Menschen (Homo sapiens), dem Schimpansen (Pan troglodytes), dem Langschwanzmakak (Macaca fascicularis) und dem Hund (canis familiaris) an.

Besonders bevorzugt bindet das erfindungsgemäße Peptid an die Lck- und Hck- SH3 von Spezies an, deren Sequenz mit der menschlichen Sequenz identisch ist.

Die Besetzung der SH3-Domäne der Lck und/oder der Hck mit dem erfindungsgemäßen Protein bzw. Peptid gemäß Sequenzprotokoll 2 ermöglicht es, eine Abstoßung von bei Patienten transplantierten Organen zu verhindern oder zumindest die Wahrscheinlichkeit einer Organabstoßung zu reduzieren.

Weiterhin können für den Organismus gefährliche Viren, wie HIV oder Hepatitis C in ihrer Wirkung geschwächt oder sogar vollkommen desaktiviert werden, da deren Effektormoleküle nicht mehr an die SH3-Domäne der Lck oder Hck binden und damit die Immunabwehr behindem bzw. außer Kraft setzen können. Eine Behandlung von mit den entsprechenden Viren assoziierten Krankheiten ist daher möglich. Beispielsweise kann bei einer HIV-1 Infektion AIDS bzw. bei einer Hepatitis C Infektion eine virusbedingte Hepatitis behandelt werden.

Das erfindungsgemäße Peptid kann als Bestandteil von in-vitro/in-vivo Aktivitätstests für Kinasen verwendet werden, die die Aufklärung von Signalwegen in der biomedizinischen Grundlagenforschung ermöglichen.

Das Immunsystem kann durch die Besetzung der Lck und/oder der Hck SH3 Domäne durch das erfindungsgemäße Peptid gemäß Seq. 2 beeinflusst werden. Wird eine Schwächung des Immunsystems hervorgerufen, kann eine Abstoßung von bei Patienten transplantierten Organen verhindert oder zumindest die Wahrscheinlichkeit einer Abstoßung nach deren Transplantation reduziert werden. Ebenfalls können Autoimmunkrankheiten wie Multiple Sklerose, systemischer Lupus erythematodes, insulinabhängiger Diabetes mellitus, rheumatische Arthritis, chronische Thyreoiditis, Spondylitis ankylosans, akute anteriore Uveitis, Goodpasteure Syndrom, Basedow Krankheit, Myasthenia gravis und Pemphigus vulgaris therapiert werden. Wird eine Stärkung des Immunsystems durch das erfindungsgemäßen Peptid gemäß Seq. 2 hervorgerufen, ist eine begleitende Therapie mit aus den Peptiden abgeleiteten Wirkstoffen bei sämtlichen Krankheiten möglich, deren Verlauf durch eine gestärkte Immunabwehr abgeschwächt werden kann (z. B. Krebs, bakterielle und virale Infektionen im Allgemeinen).

Die Erfindung umfasst auch Arzneimittel, welche das erfindungsgemäße Peptid gemäß Sequenzprotokoll 2 umfasst.

Mit den Arzneimitteln können die Krankheiten Aids oder Erkrankungen, die durch das Virus Hepatitis C bedingt sind und die oben genannten Autoimmunkrankheiten behandelt werden.

Im Hinblick auf die Verdrängung des HIV-1 Effektormoleküls Nef von der Lck SH3 Domäne wurde für PD1-R (Seq. 2) ein besonders gutes Ergebnis festgestellt.

Die erfindungsgemäßen Peptide können nach bekannten Verfahren hergestellt werden.

So kann zur Herstellung ein chemisch-synthetisches Verfahren, zum Beispiel Festphasen-Synthesen oder eine Synthese in flüssigem Medium, herangezogen werden. Bei der Festphasensynthese werden die Aminosäuren in der Abfolge der Sequenz entsprechend den Sequenzprotokollen miteinander verbunden. Die Festphasenpeptidsynthese besteht aus drei wesentlichen Schritten.

1) Aufbau der Aminosäurekette zum Peptid,

2) Spaltung des synthetisierten Peptids vom Harz und

3) Reinigung und Charakterisierung. Für die Synthese der Aminosäurekette sind unterschiedliche Kopplungsmethoden bekannt, wie Sie beispielsweise in "Beyer Walter" 22. Auflage ISBN 3-7776-0485-2 Seite 829-834 beschrieben werden.

Für die Herstellung der erfindungsgemäßen Peptide kann jede Standard-Peptidsynthese eingesetzt werden.

Das Peptid kann auch durch Expression der für sie kodierende Nucleotidsequenz, zum Beispiel in Chromosomen, Plasmiden oder anderen Informationsträgern, nackte DNA oder RNA in Organismen, in Zellen oder zellfreien Systemen, hergestellt werden.

Der erfindungsgemäße Wirkstoff und die pharmazeutische Zusammensetzung können als flüssige, halbfeste oder feste Arzneiformen und in Form von z. B. Injektionslösungen, Tropfen, Säften, Sirupe, Spray, Suspensionen, Granulaten, Tabletten, Pellets, transdermalen therapeutischen Systemen, Kapseln, Pflastern, Zäpfchen, Salben, Cremes, Lotionen, Gelen, Emulsionen oder Aerosolen vorliegen und verabreicht werden, und enthalten die erfindungsgemäßen Peptide in einer physiologisch verträglichen Form und in Abhängigkeit des Applikationsweges pharmazeutischer Hilfsstoffe, wie z. B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsmittel können beispielsweise sein: Wasser, Ethanol, 2-Propanol, Glycerin, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearät, Natriumlaurylsulfat, genießbare Öle, Sesamöl, Kokosnussöl; Erdnussöl, Sojabohnenöl, Lecitin, Natriumlactat, Polyoxyethylen- und - propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumoxid, Titanoxid, Titandioxid, Magnesiumsulfat, Zinksulfat, Calciumsulfat, Pottasche, Calciumphosphat, Dicalciumphosphat, Kaliumbromid, Kaliumjodid, Talkum, Kaolin, Pectin, Crospovidon, Agar und Bentonit.

Die Auswahl der Hilfsstoffe sowie der einzusetzenden Mengen derselben hängt davon ab, ob das Medikament oral, subkutan, parenteral, intravenös, pulmonal, intraperitoneal, transdermal, intramuskulär, nasal, buccal, rectal oder auf andere geeignete Weise appliziert werden soll. Für die orale Applikation eignen sich u. a. Zubereitungen in Form von Tabletten, Dragees, Kapseln, Granulaten, Tropfen, Säften und Sirupe, für die parenterale, topische und inhalative Applikation Lösungen, Suspensionen, leicht rekonstruierbare Pulver zur Inhalation sowie Sprays. In geeigneten perkutanen Applikationsformen kann der Wirkstoff in einem Depot in gelöster Form oder in einem Pflaster, gegebenenfalls unter Zusatz von die Hautpenetration fördernden Mitteln vorliegen. Rektal, transmucosal, parenteral, oral oder percutan anwendbar re Zubereitungsformen können die erfindungsgemäßen Peptide verzögert freisetzen.

In flüssiger Form können die erfindungsgemäßen Peptide beispielsweise intravenös, oral, als Nasenspray, subcutan, intramuskulär, inhalativ oder in oder neben das Rückenmark appliziert werden. Weiterhin können die erfindungsgemäßen Wirkstoffe mittels Salben oder Cremes aufgebracht werden.

Die erfindungsgemäßen Peptide können am Wirkort oder an anderen Orten des Organismus entstehen, nachdem Nucleinsäuren (DNA und RNA oder eine Kombination davon), die für die erfindungsgemäßen Peptide kodieren, in den Organismus eingebracht werden. Dies kann durch virale Vektoren, nackte Nucleinsäure (DNA,RNA), Plasmide, künstliche Viruspartikel, Liposomen erreicht werden, die intravenös, intranasal, oral, rectal, subcutan, intramuskulär, in oder an das Rückenmark in den Körper eingebracht werden.

Beispiele:

Protokoll entsprechend Sequenz Nr.1: Verwendet wurde eine kommerzielle Phagenbibliothek, welche die Präsentation von 12 rändomisierten Aminosäuren erlaubt (Ph.D.-12™ Phage Display Library Kit, New England Biolabs, Frankfurt). Hier ist an das Phagenhüllprotein kodierende gp3-Gen nach der Signalsequenz N-terminal die Bibliothek inseriert. Diese besteht aus $1{,}9 \times 10^9$ unabhängigen Klonen und ist so amplifiziert und konzentriert, dass in 10 µl durchschnittlich jede Sequenz in 10 Kopien vorliegt. Zur Durchführung der Selektion gegen die SH3 Domäne der menschlichen T-Zell Tyrosinkinase (Lck SH3) wurde rekombinant hergestellte Lck SH3 als Fusion mit Glutathion-S-Transferase (GST-Lck-SH3) in einer Konzentration von 100 µg/ml in Tris-Bis-Puffer (20 mM Tris-Bis, 150 mM NaCl, pH 6,5) in einer Mikrotiterplattenvertiefung immobilisiert. Hierfür wurden jeweils 200 µl Proteinlösung über Nacht bei 4°C unter sanftem Schütteln inkubiert. Nach Entfernen der Lösung wurden verbleibende Bindungsstellen der Mikrotiterplattenvertiefung mit BSA (10 mg/ml in Tris-Bis) blockiert (60 Minuten, Inkubation unter leichtem Schütteln). Danach wurden 6 Waschschritte mit 200 µl Tris-Bis durchgeführt. Für die Selektion wurden 10 µl Phagen der kommerziellen Phagenbibliothek in 100 µl Tris-Bis mit 0,1 % Tween (Tris-BisT) für 20 Minuten unter leichtem Schütteln in einer GST-Lck-SH3-beschichteten Mikrotiterplattenvertiefung inkubiert. Die nicht gebundenen Phagen wurden verworfen und anschließend 10 mal mit 200 µl Tris-BisT gewaschen. Die Elution erfolgte mit 100 µl 0,2 M Glycin-HCl, pH 2,2 für 10 Minuten unter leichtem Schütteln. Das Eluat wurde durch Zugabe von 25 µl 1M Tris-HCl, pH 9,1 neutralisiert. Zur Amplifikation der im Eluat enthaltenen Phagen wurde 9/10 des Eluats zu 20 ml E. coli-Kultur ($OD_{600}$ von 0,1) gegeben und für 5 Stunden bei 37°C unter starker Belüftung inkubiert. Anschließend wurde die Kultur 20 Minuten bei 5000 rpm zentrifugiert. Der Überstand wurde überführt und 1/6 Volumen PEG/NaCl (20% Polyethylenglycol-8000; 2,5 M NaCl) zugegeben. Der Ansatz wurde nun über Nacht auf Eis gefällt. Danach wurde 60 Minuten bei 5000 rpm zentrifugiert, der Überstand verworfen und das Pellet in 1 ml TBS (50 mM Tris-HCl, pH 7, 5, 150 mM NaCl) resuspendiert. Anschließend wurde 5 Minuten bei 10000 rpm zentrifugiert. Der Überstand wurdc überführt und 1/6 Volumen PEG/NaCl zugegeben. Der Ansatz wurde 1 Stunde auf Eis gefällt. Abschließend wurde 60 Minuten zentrifugiert und das Pellet in 100 µl TBS resuspendiert. Die so erhaltenen Phagen konnten nun in der nächsten Runde eingesetzt werden. Es wurden drei Selektionsrunden durchgeführt. Anschließend wurden willkürlich ausgewählte Phagenklone isoliert und die Aminosäuresequenz der auf diesen Phagen präsentierten Peptide. über den Umweg der DNA-Sequenzierung des im Phagengenom, kodierten Peptids bestimmt.

Peptid PD1 (HSKYPLPPLPSL) Sequenz Nr. 1 wurden am Ende der Selektion von 11 der insgesamt 18 charakterisierten Phagenklonen präsentiert, die zugehörige DNA-Sequenz ist Sequenz Nr.3

Charakterisierung der Lck-SH3 Bindungseigenschaften von PD1 (Seq.1). PD1-R (Seq.2) und PD1-R(-5)F (Seq.4)

A. "anti-Phage-ELISA" - Bestimmung der relativen Lck-SH3-Bindungsaktivität einzelner Phagenklone mit PD1 (Seq. Nr.1)

Dieser Bindungstest ist ein häufig verwendeter Test zur schnellen Bestimmung relativer Bindungsstarken selektierter Peptide, die hierbei noch physikalisch mit dem Phagen verknüpft vorliegen. Dieser Schritt dient auch zur Eliminierung von (bei dieser Methode nicht selten auftretenden) Phagenvarianten, die z. B. aufgrund ihrer Affinität zur Plastikoberfläche der verwendeten Mikrotiterplatten, angereichert werden und die somit zur Verfälschung der Ergebnisse führen können. Eine grundsätzliche Beschreibung der Methode findet sich z. B. unter Sparks et al. Methods Enzymol. 1995; 255:498-509.

Protokoll:

GST-Lck-SH3 Protein wurde in einer Konzentration von 10 µg/ml in Tris-Bis über Nacht bei 4°C in Mikrotiterplatten immobilisiert. Verbleibende Bindungsstellen wurden durch 2-stündige Inkubation mit Blockierungspuffer (Bis-Tris mit 2.% Magermilchpulver) abgesättigt.

In einer anderen Mikrotiterplatte wurden zuerst je 100 µl Suspension der zu testenden Phagenvariante mit 100 µl Blockierungspuffer für 20 Minuten vorinkubiert und erst dann für 1 Stunde zum immobilisierten GST-Lck-SH3 Protein gegeben. Nach einem Waschschritt mit Bis-Tris wurde ein Meerrettich-Peroxidase-konjugierter Antikörper zugegeben und die Platten für eine weitere Stunde inkubiert. Nach weiteren drei Waschschritten mit Bis-Tris konnten die gebundenen Phagen mittels einer Farbreaktion unter Verwendung von 3,3',5,5'-Tetramethylbenzidine/Wasserstoffperoxid nach Abstoppen mit 100 µl Schwefelsäure bei 450 nm detektiert werden (Brüsselbach S, Korn T, Völkel T, Müller R, Konterman RE. Enzyme recruitment and tumor cell killing in vitro by a secreted bispecific single-chain diabody. Tumor Targeting 1999; 4:115-123). Die Intensität der Gelbfärbung wurde mittels eines Fluostar Optima Mikrotiterplatten-Lesegerätes (BMG Labtechnologies GmbH, Offenburg) dokumentiert und gibt die relative Bindungsstärke der getesteten Phagenvariante an.

B. Fluoreszenztitration - Bestimmung von Bindungskonstanten mit PD1 und PD1-R (Seq.1 und Seq.2)

Die Bestimmung der Lck-SH3-Bindungsstärke sowie der Bindungsstärke an die SH3-Domänen aus Hck, Abl, PI3K des Peptides PD1 durch Messung der Änderung der intrinsischen Tryptophanfluoreszenz basiert auf der Wechselwirkung von PD1 mit bestimmten konservierten aromatischen Aminosäureresten (hauptsächlich Tryptophan) in der Peptidbindungstasche der SH3-Domäne. Die Messungen wurden im Wesentlichen wie bei Schweimer K, Hoffmann S, Bauer F, Friedrich U, Kardinal C, Feller SM, Biesinger B, Sticht H. Structural investigation of the bindung of a herpesviral protein to the SH3 domain of tyrosine kinase Lck. Biochemistry. 2002 Apr 23;41(16):5120-30 beschrieben unter Verwendung eines Perkin-Elmer LS 55 Fluoreszenzspektrometers bei einer Anregungswellenlänge von 290 nm und einer Emissionswellenlänge von 345 nm durchgeführt. Die Titrationskurven, die zur Bestimdung der Bindungskonstanten benötigt werden, wurden durch graduelle Zugabe von PD1 (aus einer Peptidstammlösung in Wasser) zu 1,6 ml mit 0,5 $\mu$M der freien Lck-SH3 Domäne (ohne GST-Fusion) bzw. der anderen SH3-Domänen in PBS, 1mM DDT gegeben. Nach PD1-Zugabe wurde jeweils die resultierende Fluoreszenzänderung aufgezeichnet. Durch eine Kontrolltitration (Hintergrundmessung), bei welcher die Peptidlösung gegen Wasser ersetzt wurde, konnten die Abweichungen der Fluoreszenzweite, welche durch eine sinkende SH3-Endkonzentration während der Titration hervorgerufen wurden, ausgeglichen werden. Da PD1 einen Tyrosinrest in der Peptidsequenz enthält, wurden Hintergrundmessungen durchgeführt, bei welchen pro Titrationsschritt Tyrosinamid in der gleichen Konzentration wie PD1 eingesetzt wurde. Die erhaltenen experimentellen Daten wurden unter Verwendung von Gleichung 1 angepasst, wobei $L_{ges}$ der absoluten Peptid- (Liganden) und $P_{ges}$ der absoluten SH3-(Protein) Konzentration pro Messpunkt entspricht, F ist die gemessene Fluoreszenzintensität für jede PD1-Konzentration und $F_{max}$ ist die maximale beobachtete Fluoreszenzintensität des Proteins bei Sättigung mit PD1, $F_{min}$ ist die Fluoreszenzintensität des Proteins bei Titrationsstart.

Gleichung 1:

$$F = F_{min} + \left( \frac{[P_{ges}] + [L_{ges}] + K_d}{2} - \sqrt{\frac{([P_{ges}] + [L_{ges}] + K_d)^2}{4} - [P_{ges}] * [L_{ges}]} \right) * \frac{(F_{max} - F_{min})}{P_{ges}}$$

Durch nichtlineare Regression mit $F_{max}$ und $K_d$ als angepasste Parameter konnten die experimentellen Daten an obige Gleichung angepasst werden.

C. NMR Spektroskopie - Nachweis der HIV1-Nef-Verdrängung von Lck- und Hck-SH3 durch Peptidliganden mit PD1 und PD1-R (Seq.1 und Seq.2)

Kernmagnetische (NMR) Resonanzspektren wurden bei einer Protonenfrequenz von 600 MHz bei 298 K mit einem Varian Unity INOVA Spektrometer aufgenommen. Die Bindung von Liganden (PD1, HIV1-Nef) an die Hck-SH3 Domäne wurde anhand bei Komplexbildung auftretenden, von der Ligandenkonzentration abhängigen, chemischen Verschiebungsänderungen verfolgt. Die dafür notwendigen Daten wurden durch eine Titrationsreihe heteronuklearer Einquantenkohärenz-Experimente ($^{1}$H,$^{15}$N HSQC-Experimente) unter Verwendung von $^{15}$N-markiertem Hck-SH3-Protein mit jeweils steigender Liganden-Konzentration ermittelt. Die Anfangskonzentration au Hck-SH3 lag dabei zwischen 200 und 400 $\mu$M (PBS, 1mM $\beta$-Mercaptoethanol). PD1-Stammlösungen lagen in Konzentrationen zwischen 5 und 10 mM vor. Titrationen wurden bis zu einem 4-fachen Überschuss an Peptid durchgeführt. Die HIV1 Nef-Stammlösung lag in einer Konzentration von 1,6 mM (PBS, 14 mM ß-Mercaptoethanol) vor, die entsprechenden Titrationen erfolgten bis zu einem Verhältnis von 1:1. Um eine HIV1-Nef-Verdrängung von der Hck-SH3 Domäne nachzuweisen, wurden alle dazu nötigen $^{1}$H, $^{15}$N HSQC-Experimente mit Ligandenkonzentrationen im Sättigungsbereich durchgeführt. Im Einzelnen wurde A. die freie Hck-SH3-Domäne vorgelegt und zu dieser HIV1-Nef titriert, B. ein Hck-SH3-HIV 1-Nef-Komplex vorgelegt und zu diesem Peptid PD1 titriert und C. die freie Hck-SH3 -Domäne vorgelegt und zu dieser Peptid PD1 titriert.

D. PepSpot-Membran - Bestimmung der relativen Lck-Bindungsaktivitäten verschiedenster PD1-R-Substitutionsvarianten; Auffinden von PD1-R-(-5)F (Seq. 4)

Eine andere Methode, Protein-Protein-Wechselwirkungen zu untersuchen, ist die Benutzung so genannter PepSpot-Membranen. Sie basiert auf der Synthese von immobilisierten Peptiden auf aktivierten Membrane und kann z. B. zur Optimierung der Bindungseigenschaften bereits identifizierter Liganden durch Substitutionsanalyse verwendet werden. Nach der Inkubation der Membran mit dem zu untersuchenden Protein (hier Lck-SH3) und der anschließenden Detektion wird anhand der Spotfärbung eine Interaktion des Proteins mit den entsprechenden immobilisierten Peptiden nachgewiesen. Die Stärke der Spotintensitäten ist ein Maß für die Stärke der Bindung. Eine zusammenfassende Übersicht findet sich bei Reinecke U., Volkmer-Engert R., Schneider-Mergener J. Applikation of peptide arrays prepared by the SPOT-technology. Current Opinion in Biotechnology 2001, 12:59-64.

Für den Immunoassay wurde die PepSpot-Membran über Nacht in 20 ml Blocking Puffer geblockt. Nach einmaligem Waschen mit T-TBS für 10 min wurde die Membran für 3 h mit 0,5 μg/ml SH3 Protein in 20 ml Blocking Puffer inkubiert. Danach wurde die Membran dreimal für je 10 min mit T-TB S gewaschen. Nach anschließender Inkubation mit 5 μl des Antikörpers Anti-GST HRP conjugated (Novus Biologicals, Inc., Littleton, USA) in 20 ml Blocking Puffer für je 2 h erfolgte dreimaliges Waschen mit T-TBS für je 20 min. Alle bisherigen Schritte wurden unter Schütteln durchgeführt. Die Detektion erfolgte am Gel Doc 2000 Gerät (Bio-Rad, München) 1 min nach Zugabe des Substrates Chemiluminescence Western Blotting Reagent (Pierce.Biotechnology, Inc., Rockford, USA). Die Spot-Intensitäten wurden mit der QuantityOne Software (Bio-Rad, München) ausgewertet Die Analyse der Spot-Intensitäten erfolgte nach Subtraktion des Hintergrundes.

E. Ergebnisse:

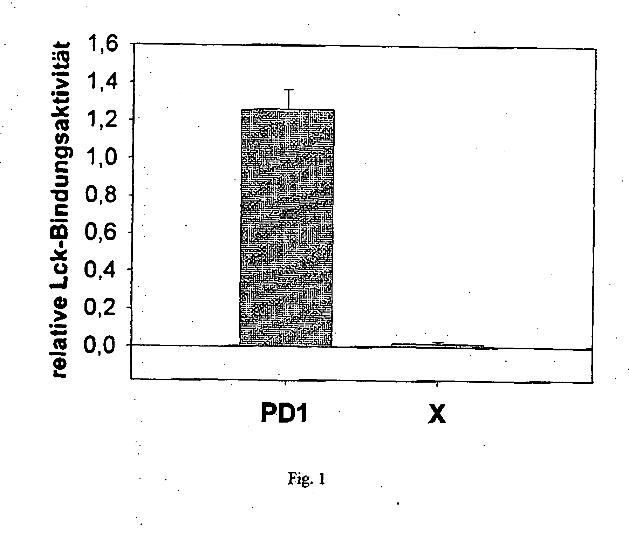

Fig. 1 zeigt Bestimmung der relativen Lck-SH3-Bindungsaktivität des Peptids PD1 (Seq. 1) mittels "anti-Phage"-ELISA.

X ist hier das nicht bindende Peptid HTLQIPQHATSF

Es wurde die relative Bindungsaktivität von PD1-präsentierenden Phagenvarianten an die Lck-SH3 bestimmt. Gezeigt sind Mittelwert und Standardabweichung aus insgesamt 22 Einzelexperimenten. Zum Vergleich ist die Lck-Bindungsaktivität eines nicht bindenden Peptides HTLQIPQHATSF aufgetragen. Hierzu wurden 7 Einzelexperimente durchgeführt. Unter den Balken sind die Namen der Peptide angegeben, die von den jeweiligen, selektierten Phagen präsentiert werden.

Tabelle 1: Bestimmung der SH3-PD1-Bindungskonstanten (Seq. 1).

Bestimmung der Bindungskonstanten ($K_D$) für den Komplex aus Peptid PD1 und Lck-SH3 sowie weiterer SH3-Domänen durch Fluoreszenztitration. Jedes Experiment wurde zweimal durchgeführt.

| SH3 Domäne | $K_d$ (μM) |
|---|---|
| Lck | 48.66 ± 1.65 |
| Hck | 0.23 ± 0.03 |
| PI3K | 112.14 ± 10.08 |
| Abl | >> 300 |

Abkürzungen: Hck: hematopoethische Zell-spezifische Kinase, PI3K: Phosphoinositol-3-Kinase, Abl: Abelson-Tyrosinkinase

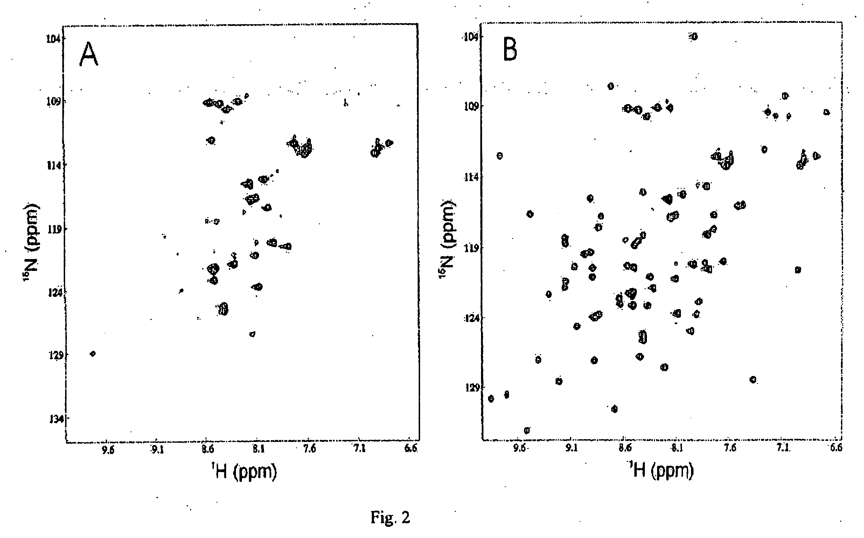

Fig.2 zeigt den Nachweis der HIV1-Nef-Verdrängung von Hck-SH3 durch PD1 mittels NMR-Spektroskopie.

Alle nötigen $^1$H, $^{15}$N HSQC-Experimente wurden mit Ligandenkonzentrationen im Sättigungsbereich durchgeführt. Zu erkennen sind jeweils die Signale der $^{15}$N-markierten Hck-SH3-Domäne. (A) Die freie Hck-SH3-Domäne wurde vorgelegt und zu dieser HIV1-Nef im Verhältnis 1:1 titriert. Dargestellt ist der Endpunkt der Titration. (B) Zu dem Hck-SH3-HIV 1-Nef-Komplex aus (A) wurde das Peptid PD1 zutitriert. Die daraus entstandenen Signale entsprechen im Wesentlichen denen eines Hck-SH3-PD1-Komplexspektrums.

Tabelle 2: Bestimmung der SH3-PD1-R-Bindungskonstanten

Bestimmung der Bindungskonstanten ($K_D$) für den Komplex aus Peptid PD1-R und Lck-SH3 sowie weiterer SH3-Domänen durch Fluoreszenztitration. Jedes Experiment wurde zweimal durchgeführt.

| SH3 Domäne | $K_d$ (μM) |
|---|---|
| Lck | 23.11 ± 1.97 |

(fortgesetzt)

| SH3 Domäne | $K_d$ (µM) |
|---|---|
| Hck | 0.47 ± 0.02 |
| PI3K | 13.12 ± 0.48 |
| Abl | >> 300 |

Abkürzungen: Hck: hematopoethische Zell-spezifische Kinase, PI3K: Phosphoinositol-3-Kinase, Abl: Abelson-Tyrosinkinase

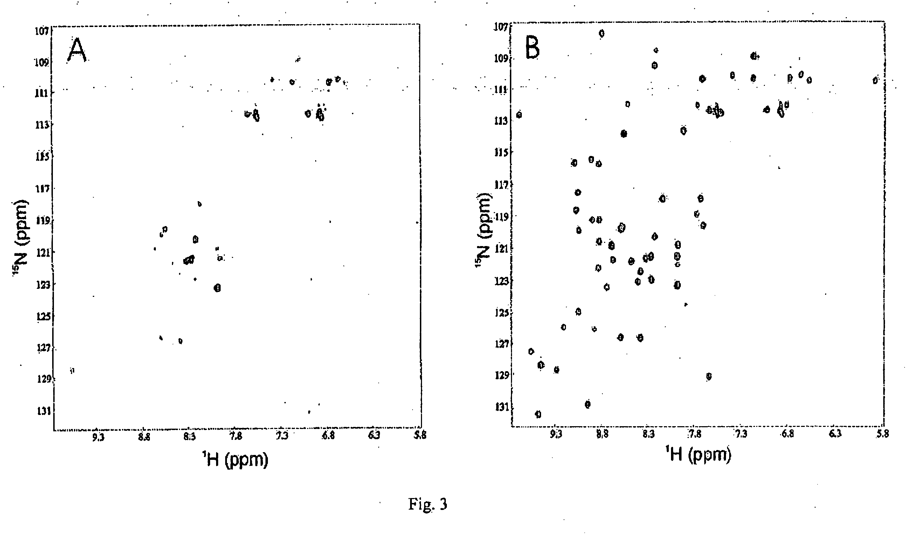

Fig. 3 zeigt den Nachweis der HIV-1 Nef-Verdrängung von Lck-SH3 durch PD1-R mittels NMR-Spektroskopie. Alle nötigen $^1$H, $^{15}$N HSQC-Experimente wurden mit Ligandenkonzentrationen im Sättigungsbereich durchgeführt. Zu erkennen sind jeweils die Signale der $^{15}$N-markierten Lck-SH3-Domäne. (A) Die freie Lck-SH3-Domäne wurde vorgelegt und zu dieser HIV1-Nef im Verhältnis 1:1 titriert. Dargestellt ist der Endpunkt der Titration. (B) Zu dem Lck-SH3-HIV1-Nef-Komplex aus (A) wurde das Peptid PD1-R zutitriert. Die daraus entstandenen Signale entsprechen im Wesentlichen denen eines Lck-SH3-PD1-Komplexspektrums.

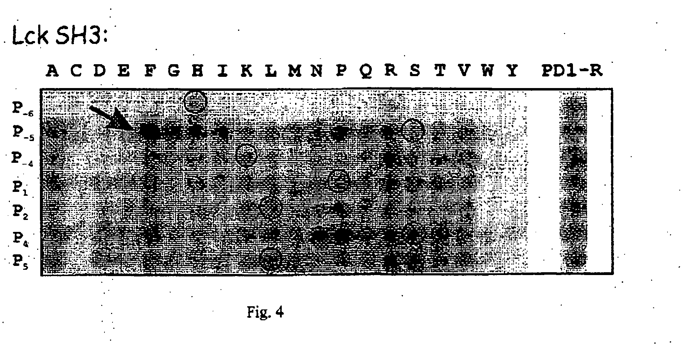

Fig. 4 zeigt die Substitutionsanalyse des Peptids PD1-R; Peptid PD1-R-(-5)F (Seq. 4) zeigt die stärkste Bindung

Insgesamt 7 der 12 Aminosäuren des Peptids PD1-R wurden gegen alle anderen natürlichen Aminosäuren unter Verwendung einer PepSpot-Membran ausgetauscht. Die Buchstaben oberhalb der Membran zeigen die Aminosäuresubstitutionen der jeweiligen Spalten im Einbuchstabencode an. Links ist die jeweilige PD1-R Position nach Lim et al. (Lim WA, Richards FM, Fox RO. Structural determinants of peptide-binding orientation and of sequence specificity in SH3 domains, Nature, 1994 Nov 24; 372(6504):375-9) zu sehen. Die Zuordnung zu den Aminosäurepositionen (angegeben als Aminosäure im Einbuchstabencode und der zugehörige Position im Peptid) gemäß PD1-R (Seq. 2) ist wie folgt: $P_6$ ist H1; $P_{-5}$ ist S2, $P_4$ ist K3, $P_1$ ist P8; $P_2$ ist L9, $P_4$ ist S11 und $P_5$ ist L12. Als Kontrollen sind rechts die Spots des Peptids PD1-R zu sehen. Die Lage von PD1-R Ausgaugspeptiden innerhalb des Substitutionsexperimentes ist durch Kreise gekennzeichnet. Das Peptid PD1-R(-5)F (Seq. 3) mit der Sequenz HFKRPLPPPLPSL zeigt die stärkste Bindung an die Lck-SH3 und ist durch einen Pfeil gekennzeichnet.

Sequenzprotokolle:

PD1 entspricht Sequenz_1
PD1-R entspricht Sequenz_2
PD1-R(-5) entspricht Sequenz_4

Sequenz Nr. 3 entspricht DNA-Sequenz von Sequenz Nr.1

Nutzen der Erfindung:

Im Speziellen kann die Pathogenität solcher viraler Erreger geschwächt werden, die direkt Effektormoleküle, welche an die SH3-Domäne von Lck (Hck) binden, besitzen. Hierzu gehören z. B. die menschlichen Krankheitserreger HIV-1 (Nef-Protein) und Hepatitis C (NS5A Protein).

Krankheiten: HIV1, Hepatitis C und Autoimmunkrankheiten

Überraschenderweise wurde für PD1 eine hochaffine Bindung an die SH3-Domäne der hematopoethischen Zell-spezifischen Kinase (Hck) festgestellt (siehe Tab.1, $K_D$ ca. 0,2 µM). Die Selektivität der PD1-Bindung an Lck und Hck könnte durch dessen schlechte Bindungseigenschaften an die nicht zum Src-Typ gehörenden Kinasen PI3K und Abl nachgewiesen werden (Tab1, $K_D$s von ca.129 und >>300 µM).

Lck und Hck sind beides Modulatoren der Immunantwort, wirken jedoch in unterschiedlichen Zelltypen. Lck liegt ausschließlich in T- und Killer-Zellen vor, Hck wird in Monocyten/Makrophagen (Ziegler et al. Novel protein-tyrosine kinase gene (hck) preferentially expressed in cells of hematopoietic origin Mol Cell Biol. 1987 Jun;7(6):2276-85) aber auch in Neutrophilen, Eosinophilen, dendritischen Zellen und Osteoklasten exprimiert.

Die menschliche hematopoetische-Zell Tyrosinkinase (Hck) ist in einer Vielzahl von Signalwegen involviert Ihr wird u. a. eine wichtige Rolle während der Phagocytose (Suzuki et al. Differential involvement of Src family kinases in Fc gamma receptor-mediated phagocytosis. J. Immunol. 2000 Jul 1; 165(1):473-82), bei der FcγRII-Rezeptor (Ghazizadeh et al. Physical and functional association of Src-related protein tyrosine kinases with Fc gamma RII in monocytic THP-1 cells, J. Biol. Chem. 1994 Mar 25; 269(12):8878-84) bzw. Integrin-vermittelten Signalübertragung (Mocsai et al. Adhesion-dependent degranulation of neutrophils requires the Src family kinases Fgr and Hck. J. Immunol. 1999 Jan 15; 162(2):1120-6) und der Chemotaxis von Monocyten (Kumar et al. Soluble E-selectin induces monocyte chemotaxis through Src family tyrosine kinases, J. Biol. Chem. 2001 Jun 15; 276(24):21039-45) zugeschrieben.

Peptidliganden der Hck-SH3-Domäne können somit ebenfalls zur Unterdrückung der Immunantvvort eingesetzt werden, wie es bei Organtransplantationen notwendig ist.

Wie auch bei der Lck kann die Komplexbildung von exogenen oder transformierten zelleigenen Liganden mit der SH3-Domäne der Hck zu deren Deregulierung und somit zu schweren Störungen von Abläufen innerhalb der normalen Immunabwehr der betroffenen Zelltypen kommen. Die Interaktion des Nef-Proteins aus HIV-1 mit der SH3-Domäne der Hck gehört dabei zu einem der stabilsten SH3-Ligandenkomplexe, die bislang beschrieben wurden (Lee et al. A single amino acid in the SH3 domain of Hck determines its high affinity and specificity in binding to HIV-1 Nef protein. EMBO J. 1995 Oct 16;14(20):5006-15). Es gibt Hinweise, dass die Interaktion von Nef mit Hck-SH3 an der Ausprägung des durch HIV-1 verursachwen Krankheztsbildes AIDS beteiligt ist (Hanna et al. The pathogenicity of human immunodeficiency virus (HIV) type 1 Nef in CD4C/HIV transgenic mice is abolished by mutation of its SH3-binding domain, and disease development is delayed in the absence of Hck. J Virol. 2001 Oct;75(19):9378-92).

Die ermittelte Bindungsstärke des PD1-Hck-SH3 (Tab. 1) liegt in der gleichen Größenordnung, wie er für einen Komplex aus Hck-SH3 mit dem HIV-1 Nef Proteins bekannt ist Durch NMR-Experimente (Abb. 2) wurde nachgewiesen, dass - zumindest *in vitro* - eine effektive Verdrängung des viralen Nef-Proteins von der Hck-SH3 Domäne und somit eine Therapie von AIDS möglich ist.

**Patentansprüche**

1. Peptid gemäß der Sequenz Nr. 2.

2. Arzneimittel,
   **dadurch gekennzeichnet,**
   **dass** es das Peptid gemäß Anspruch 1 enthält.

3. Arzneimittel nach Anspruch 2,
   **dadurch gekennzeichnet, dass** es ein Mittel zur Linderung und/oder Heilung der Krankheiten AIDS, Hepatitis, Multiple Sklerose, systemischer Lupus erythematodes, Diabetes, rheumatische Arthritis, chronische Thyreoiditis, Spondylitis ankylosans, anteriore Uveitis, Goodparteure Syndrom, Basedow Krankheit, Myasthenia gravis, Krebs und Pemphigus vulgans ist.

4. Lck-SH3 Komplex
   **dadurch gekennzeichnet,**
   **dass** sein Komplexpartner das Peptid nach Anspruch 1 ist.

5. Lck-SH3.Komplex nach Anspruch 4,
   **dadurch gekennzeichnet**
   das er ein Komplex einer SH3-Domäne vom Menschen, von der Maus, der Ratte, dem Hund, dem Nachtaffen, dem Schimpansen oder einer Spezies ist, welche eine SH3-domäne besitzt, welche mit der des Menschen identisch ist.

6. Hck-SH3 Komplex
   **dadurch gekennzeichnet,**
   **dass** sein Komplexpartner das Peptid nach Anspruch 1 ist.

**7.** Hck-SH3 Komplex nach Anspruch 6,
**dadurch gekennzeichnet**
das er ein Komplex einer SH3-Domäne vom Menschen, vom Schimpansen, dem Langschwanzmakak, dem Hund oder einer Spezies ist, welche eine SH3-Domäne besitzt, welche mit der des Menschen identisch ist.

## Claims

**1.** Peptide according to sequence No 2.

**2.** Drug,
**characterised in that**
it contains the peptide according to claim 1.

**3.** Drug according to claim 2,
**characterised in that** it is a preparation for alleviating and/or curing AIDS, hepatitis, multiple sclerosis, systemic lupus erythematosus, diabetes, rheumatic arthritis, chronic thyroiditis, spondylitis ankylosans, anterior uveritis, good pasteur's syndrome, basedow disease, myasthenia gravis, cancer and pemphigus vulgaris.

**4.** Lck-SH3 complex,
**characterised in that**
its complex partner is the peptide according to claim 1.

**5.** Lck-SH3 complex according to claim 4,
**characterised in that**
it is a complex of an SH3 domain of people, mice, rats, dogs, owl monkeys, chimpanzees or species, which have an SH3 domain identical to that of people.

**6.** Hck-SH3 complex,
**characterised in that**
its complex partner is the peptide according to claim 1.

**7.** Hck-SH3 complex according to claim 6,
**characterised in that**
it is a complex of an SH3 domain of people, chimpanzees, long tailed macaques, dogs or a species, which have an SH3 domain identical to that of people.

## Revendications

**1.** Peptide selon la séquence n° 2.

**2.** Médicament,
**Caractérisé**
**en ce qu'**il contient le peptide selon la revendication 1.

**3.** Médicament selon la revendication 2,
**caractérisé en ce qu'**il s'agit d'un agent pour le soulagement et/ou le traitement de maladies telles que SIDA, hépatite, sclérose en plaques, lupus érythémateux systémique, diabète, arthrite rhumatoïde, thyroïdite chronique, spondylite ankylosant, uvéite antérieure, syndrome de Goodpasture, maladie de Basedow, myasthénie gravis, cancer et pemphigus vulgaire,

**4.** Complexe de Lck-SH3,
**caractérisé en ce**
**que** son partenaire de complexe est le peptide selon la revendication 1.

**5.** Complexe de Lck-SH3 selon la revendication 4,
**caractérisé en ce**

**qu'**il est un complexe d'un domaine SH3 d'être humain, de souris, de rat, de chien, de singe de nuit, de chimpanzé ou d'une espèce qui possède un domaine SH3 qui est identique à celui de l'être humain.

6. Complexe de Lck-SH3,
   **caractérisé en ce**
   **que** son partenaire de complexe est le peptide selon la revendication 1.

7. Complexe de Lck-SH3 selon la revendication 6,
   **caractérisé en ce**
   **qu'**il est un complexe d'un domaine SH3 d'être humain, de chimpanzé, de macaque à longue queue, de chien ou d'une espèce qui possède un domaine SH3 qui est identique à celui de l'être humain.

Fig. 1

Fig. 2

Fig. 3

Lck SH3:

A C D E F G H I K L M N P Q R S T V W Y   PD1-R

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **Poghosyan Z. et al.** Phosphorylation-dependent interactions between ADAM15 cytoplasmic domain and Scr family protein-tyrosine kinase. *J Biol Chem.,* 15. Februar 2002, vol. 277 (7), 4999-5007
- **von Kristian Schweimer et al.** Structural Investigation of the Binding of a Herpesviral Protein to the SH3 Domain of Tyrosine Kinase Lck. *Biochemistry,* 2002, vol. 41, 5120-5230
- **Sparks et al.** *Methods Enzymol.,* 1995, vol. 255, 498-509
- **Brüsselbach S ; Korn T ; Völkel T ; Müller R ; Konterman RE.** Enzyme recruitment and tumor cell killing in vitro by a secreted bispecific single-chain diabody. *Tumor Targeting,* 1999, vol. 4, 115-123
- **Schweimer K ; Hoffmann S ; Bauer F ; Friedrich U ; Kardinal C ; Feller SM ; Biesinger B ; Sticht H.** Structural investigation of the bindung of a herpesviral protein to the SH3 domain of tyrosine kinase Lck. *Biochemistry,* 23. April 2002, vol. 41 (16), 5120-30
- **Reinecke U. ; Volkmer-Engert R. ; Schneider-Mergener.** J. Applikation of peptide arrays prepared by the SPOT-technology. *Current Opinion in Biotechnology,* 2001, vol. 12, 59-64
- **Lim WA ; Richards FM ; Fox RO.** Structural determinants of peptide-binding orientation and of sequence specificity in SH3 domains. *Nature,* 24. November 1994, vol. 372 (6504), 375-9
- **Ziegler et al.** Novel protein-tyrosine kinase gene (hck) preferentially expressed in cells of hematopoietic origin. *Mol Cell Biol.,* Juni 1987, vol. 7 (6), 2276-85
- **Suzuki et al.** Differential involvement of Src family kinases in Fc gamma receptor-mediated phagocytosis. *J. Immunol.,* 01. Juli 2000, vol. 165 (1), 473-82
- **Ghazizadeh et al.** Physical and functional association of Src-related protein tyrosine kinases with Fc gamma RII in monocytic THP-1 cells. *J. Biol. Chem.,* 25. Marz 1994, vol. 269 (12), 8878-84
- **Mocsai et al.** Adhesion-dependent degranulation of neutrophils requires the Src family kinases Fgr and Hck. *J. Immunol.,* 15. Januar 1999, vol. 162 (2), 1120-6
- **Kumar et al.** Soluble E-selectin induces monocyte chemotaxis through Src family tyrosine kinases. *J. Biol. Chem.,* 15. Juni 2001, vol. 276 (24), 21039-45
- **Lee et al.** A single amino acid in the SH3 domain of Hck determines its high affinity and specificity in binding to HIV-1 Nef protein. *EMBO J.,* 16. Oktober 1995, vol. 14 (20), 5006-15
- **Hanna et al.** The pathogenicity of human immunodeficiency virus (HIV) type 1 Nef in CD4C/HIV transgenic mice is abolished by mutation of its SH3-binding domain, and disease development is delayed in the absence of Hck. *J Virol.,* Oktober 2001, vol. 75 (19), 9378-92